(19) Europäisches Patentamt
European Patent Office
Office européen des brevets

(11) **EP 4 296 598 A1**

(12) **EUROPEAN PATENT APPLICATION**
published in accordance with Art. 153(4) EPC

(43) Date of publication:
**27.12.2023 Bulletin 2023/52**

(21) Application number: **22775420.7**

(22) Date of filing: **17.03.2022**

(51) International Patent Classification (IPC):
**F27B 1/02** (2006.01)    **F27D 7/02** (2006.01)
**F24H 3/00** (2022.01)

(52) Cooperative Patent Classification (CPC):
**F24H 3/00; F27B 1/02; F27D 7/02**

(86) International application number:
**PCT/JP2022/012436**

(87) International publication number:
**WO 2022/202632 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.03.2021   JP 2021050325**

(71) Applicant: **FUJIFILM Corporation**
**Tokyo 106-8620 (JP)**

(72) Inventors:
• **YOSHIDA, Tetsuya**
  **Ashigarakami-gun, Kanagawa 258-8577 (JP)**
• **KATO, Nobuhiko**
  **Ashigarakami-gun, Kanagawa 258-8577 (JP)**
• **YODOGAWA, Satoshi**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **HEAT TREATMENT DEVICE AND METHOD FOR MANUFACTURING HEAT TREATMENT OBJECT**

(57) The present disclosure provides a heating treatment apparatus including a container that includes a first chamber and a second chamber which are connected to each other via a sectionalizing valve and an air supply port and an air exhaust port through which a heated gas flows and a manufacturing method of a heating treatment object.

FIG. 1

**Description**

**BACKGROUND OF THE INVENTION**

1. Field of the Invention

The present disclosure relates to a heating treatment apparatus and a manufacturing method of a heating treatment object.

2. Description of the Related Art

A heating treatment apparatus is widely used in many applications such as drying and crosslinking of a heating treatment target object. As such a heating treatment apparatus, a shelf plate type heating treatment apparatus (oven) is known and is disclosed in, for example, WO2014/141877A.

**SUMMARY OF THE INVENTION**

In a case of taking out a heating treatment object (that is, an object obtained through heating treatment) from the heating treatment apparatus, a technique of suppressing mixing of foreign substances with the heating treatment object is required.

The present disclosure is devised in view of such circumstances, and an object to be achieved by an embodiment of the present disclosure is to provide a heating treatment apparatus in which a heating treatment object can be taken out while suppressing mixing with foreign substances.

An object to be achieved by another embodiment of the present disclosure to provide a manufacturing method of a heating treatment object using the heating treatment apparatus.

The present disclosure includes the following aspects.

<1> A heating treatment apparatus comprising a container that includes a first chamber and a second chamber which are connected to each other via a sectionalizing valve and an air supply port and an air exhaust port through which a heated gas flows.

<2> The heating treatment apparatus according to <1>, in which the container has a tubular shape.

<3> The heating treatment apparatus according to <1> or <2>, further comprising a temperature adjusting member that is disposed on an outer side of the container and that is detachably attached to the heating treatment apparatus.

<4> The heating treatment apparatus according to <3>, in which the temperature adjusting member includes at least one of a jacket type heater or a metal member.

<5> The heating treatment apparatus according to <4>, in which the metal member is made of stainless steel or is made of aluminum.

<6> The heating treatment apparatus according to any one of <1> to <5>, in which a holding member that holds a heating treatment target object is provided between the air supply port and the air exhaust port.

<7> The heating treatment apparatus according to <6>, in which the holding member is a porous plate, a mesh plate, a punching metal, or a breathable bag.

<8> The heating treatment apparatus according to any one of <1> to <7>, in which the sectionalizing valve is a split butterfly valve.

<9> A manufacturing method of a heating treatment object comprising:

a step of accommodating a heating treatment target object in the container of the heating treatment apparatus according to any one of <1> to <8>; and
a step of flowing the heated gas into the container via the air supply port and the air exhaust port.

<10> The manufacturing method of a heating treatment object according to <9>, in which the heating treatment apparatus further includes a temperature adjusting member, and the manufacturing method further comprises a step of heating the container with the temperature adjusting member.

<11> The manufacturing method of a heating treatment object according to <10>, in which the temperature adjusting member includes stainless steel and a heater that heats the stainless steel.

<12> The manufacturing method of a heating treatment object according to <10> or <11>, in which at least a part of the step of flowing the heated gas and at least a part of the step of heating the container are simultaneously performed.

<13> The manufacturing method of a heating treatment object according to any one of <9> to <12>, in which the heating treatment target object is a polymer porous substance of collagen or gelatin.

<14> The manufacturing method of a heating treatment object according to any one of <9> to <13>, in which the heated gas is an inert gas.

<15> The manufacturing method of a heating treatment object according to any one of <9> to <14>, further comprising a step of collecting a heating treatment object by closing the sectionalizing valve after the step of flowing the heated gas.

According to one embodiment of the present disclosure, the heating treatment apparatus in which the heating treatment object can be taken out while suppressing mixing with foreign substances is provided.

According to another embodiment of the present disclosure, the manufacturing method of a heating treatment object using the heating treatment apparatus is provided.

**BRIEF DESCRIPTION OF THE DRAWINGS**

Fig. 1 is a schematic cross sectional view showing an example of a heating treatment apparatus.

Fig. 2 is a schematic cross sectional view showing an example of a state where a first chamber and a second chamber are separated from each other.

Fig. 3 is a schematic cross sectional view showing an example of a state where a sectionalizing valve is opened.

Fig. 4 is a schematic cross sectional view showing another example of the heating treatment apparatus.

Fig. 5 is a schematic cross sectional view showing a still another example of the heating treatment apparatus.

Fig. 6 is a schematic cross sectional view showing a still another example of the heating treatment apparatus.

Fig. 7 is a schematic cross sectional view showing a still another example of the heating treatment apparatus.

**DESCRIPTION OF THE PREFERRED EMBODIMENTS**

Hereinafter, details of a heating treatment apparatus and a manufacturing method of a heating treatment object according to the embodiment of the present disclosure will be described.

In the present disclosure, a numerical range indicated by using "to" means a range including numerical values described before and after "to" as a minimum value and a maximum value, respectively.

In numerical ranges that are described stepwise in the present disclosure, an upper limit value or a lower limit value described in a certain numerical range may be replaced with an upper limit value or a lower limit value of another stepwise numerical range. In addition, in the numerical ranges described in the present disclosure, the upper limit value or the lower limit value described in the certain numerical range may be replaced with values shown in Examples.

In the present disclosure, a combination of two or more preferred aspects is a more preferred aspect.

In the present disclosure, in a case where there are a plurality of types of substances corresponding to each component, the amount of each component means a total amount of the plurality of types of substances, unless stated otherwise.

In the present disclosure, the term "step" includes not only an independent step but also includes a step that cannot be clearly distinguished from other steps but still achieves a desired effect of the step.

In the present disclosure, an amino acid sequence of a polypeptide is expressed using one-letter notation (for example, "G" in a case of a glycine residue) or three-letter notation (for example, "Gly" in a case of a glycine residue) that is well known in the technical field in some cases.

In the present disclosure, "%" related to the amino acid sequence of the polypeptide is based on the number of amino acid (or imino acid) residues unless stated otherwise. In the present invention, "identity" related to amino acid sequences of two types of polypeptides to be compared refers to a value calculated through the following equation.

Comparison (alignment) of a plurality of polypeptides is made through a conventional method such that the number of the same amino acid residues is the largest.

$$\text{Identity (\%)} = \{(\text{Number of same amino acid residues})/(\text{Alignment length})\} \times 100$$

The drawings referred to in the following description are exemplarily and schematically shown, and the present disclosure is not limited to the drawings. The same reference numerals indicate the same components. In addition, the reference numerals in the drawings are omitted in some cases.

<Heating Treatment Apparatus>

The heating treatment apparatus according to the embodiment of the present disclosure comprises a container that includes a first chamber and a second chamber which are connected to each other via a sectionalizing valve and an air supply port and an air exhaust port through which a heated gas flows. In the preferred aspect, the heating treatment apparatus may include the air supply port disposed in the first chamber and the air exhaust port disposed in the second chamber.

As the heating treatment apparatus, a shelf plate type heating treatment apparatus (oven) is known. In a case of the shelf plate type heating apparatus, after heat treatment is performed on a heating treatment target object to obtain a heating treatment object, a door is opened in order to take out the heating treatment object. In this case, the heating treatment object is exposed to the external environment

On the contrary, the heating treatment apparatus according to the embodiment of the present disclosure is separated into the first chamber and the second chamber which are in a state where the container is sealed with the sectionalizing valve, after a heating treatment target object is accommodated in the container that includes the first chamber and the second chamber, which are connected to each other via the sectionalizing valve, and a gas heated in the container is flowed to perform heating treatment. Then, after a chamber that accommodates a heating treatment object and that is sealed with the sectionalizing valve (for example, the first chamber) is connected to an external space (for example, other containers, apparatuses, rooms, or the like) via the sectionalizing valve, the heating treatment object can be taken out to the outside from the chamber (for example, the first chamber) by opening the sectionalizing valve. For this reason, the heating treatment object can be taken out from the container while suppressing mixing with foreign substances.

As described above, since mixing of foreign substances (for example, water, bacteria, or the like) can be suppressed, the heating treatment apparatus according to the embodiment of the present disclosure is suitable for moisture content maintaining, sterilizing, or the like of a heating treatment object. In particular, in a case where the heating treatment object is a bioaffinity polymer such as collagen and gelatin or a polymer porous crosslinked substance thereof, since taking out the heating treatment object in an aseptic state is advantageous and connection to the external space is possible in the aseptic state via the sectionalizing valve instead of a pass box, workability is high. Further, since the heating treatment object is accommodated in the chamber sealed with the sectionalizing valve, storing and transporting are easy while suppressing mixing with foreign substances.

The heating treatment is not particularly limited, and examples thereof include drying, crosslinking, humidity control, reaction, and the like.

A direction in which the heating treatment apparatus shown in the drawings is described is irrelevant to an actual direction in which the heating treatment apparatus is provided. For example, the heating treatment apparatus may be provided along a direction (that is, a machine direction) in which an aeration direction (a longitudinal direction of the container) is along a vertical direction or may be provided in a direction (that is, a lateral direction) in which the aeration direction in the container is perpendicular to the vertical direction.

[Container]

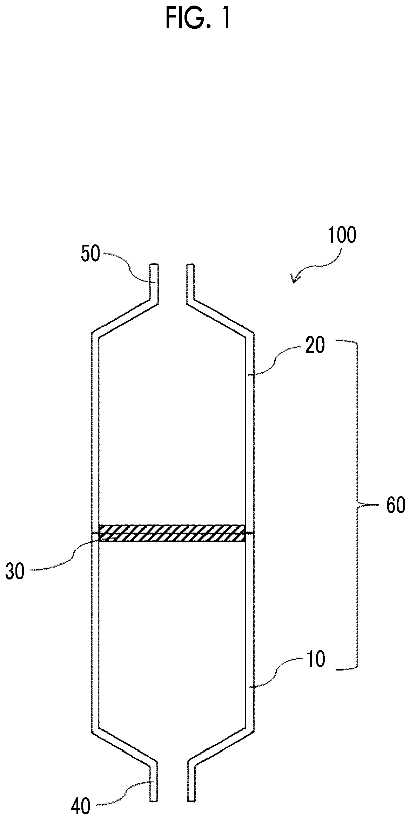

The container includes the container that includes the first chamber and the second chamber which are connected to each other via the sectionalizing valve and the air supply port and the air exhaust port through which a heated gas flows. For example, in a container 60 of a heating treatment apparatus 100 shown in Fig. 1, a first chamber 10 is provided with an air supply port 40, a second chamber 20 is provided with an air exhaust port 50, and the first chamber 10 and the second chamber 20 are connected to each other via a sectionalizing valve 30.

A material for the container is not particularly limited, but a metal is preferable from a perspective of pressure resistance. For example, the container may be made of stainless steel. In a case where the container is made of a metal, since pressure resistance is high and a gas heated in the container at a high pressure can be flowed, heating treatment can be performed on a heating treatment target object for a shorter period of time.

The shape of the container is not particularly limited, but is preferably a tubular shape, and a cross section orthogonal to a longitudinal direction of a tube may be any one of a circle, an ellipse, a rectangle, a trapezoid, or the like. The shape of the container may be, for example, a cylindrical shape, an elliptical cylindrical shape, or the like and is more preferably a cylindrical shape.

In a case where the container has a tubular shape, since a filling proportion of a heating treatment target object can be increased, for example, compared to a shelf plate type apparatus, heating treatment can be efficiently performed.

Further, by increasing the filling proportion, a decrease in size can be achieved, for example, compared to the shelf plate type apparatus. In a case of the shelf plate type apparatus, since the shelf plate type apparatus is a type that comprises a control device inside, the heating treatment apparatus according to the embodiment of the present disclosure is effective from a perspective of a decrease in size compared to such a heating treatment apparatus. Accordingly, handling (for example, storing, transporting, application to the next step (for example, cooling, freezing, filling of a hopper, or the like) or the like) of the heating treatment apparatus becomes even easier. The filling proportion is a proportion (% by volume) of the volume of the heating treatment target object with respect to the internal capacity of the container.

In addition, in a case where the container has a tubular shape, since a flow of a heated gas flowing in the container can be more uniform, it is even easier to uniformly heat a heating treatment target object.

Further, in a case where the container has a tubular shape, since pressure resistance is high and a gas heated in the container at a high pressure can be flowed, heating treatment can be performed on the heating treatment target object for a shorter period of time due to an increase in the supply amount of heat and the flow rate of the heated gas. In a case of drying the heating treatment target object, since the flow rate of a carrier gas (that is, the heated gas) increases, removal of a liquid can be further promoted.

From a perspective of uniformly heating a heating treatment target object, in a case where the container has a tubular shape, a ratio (aspect ratio) of a maximum length (that is, a maximum value of a straight line connecting any two points of an outline) of the outline of the cross section perpendicular to the height direction with respect to the length of a tube of a portion that accommodates the heating treatment target object in the aeration direction (height direction) is preferably 0.5 to 3.0 and is more preferably 1.0 to 2.5. The maximum length of the outline of the cross section perpendicular to the height direction is, for example, a diameter of a circle in a case where the container has a cylindrical shape, the major axis of an ellipse in a case where the container has an elliptical shape, and a maximum diagonal line in a case of a rectangle.

The maximum length of the outline of the container is not particularly limited, may be the same as the diameter of the sectionalizing valve, or may be larger than the diameter of the sectionalizing valve.

The number of air supply ports and the number of air exhaust ports are not particularly limited and may be selected as appropriate depending on design of the heating treatment apparatus or the like.

The positions of the air supply port and the air exhaust port are not particularly limited and may be selected as appropriate depending on design of the heating treatment apparatus or the like. In addition, depending on the design of the heating treatment apparatus or the like, which chamber accommodates a heating treatment target object may be selected, only the first chamber or the second chamber may accommodate the heating treatment target object, or both may accommodate the heating treatment target object.

The container may have one or more chambers connected to each other via the sectionalizing valve other than the first chamber and the second chamber.

In addition, the container may include a supply port for accommodating a heating treatment target object inside. Accordingly, in a state where the first chamber and the second chamber are connected to each other via the sectionalizing valve, the heating treatment target object can be accommodated inside the container.

[Sectionalizing Valve]

In the present disclosure, the term "sectionalizing valve" means, in a case of separating the two chambers connected to each other via the sectionalizing valve, a valve that can sectionalize the two chambers in a sealed state into two respectively. "Sectionalizing the chambers in a sealed state" means sectionalizing the chambers in a closed state and preferably sectionalizing the chambers in a hermetically sealed state. In addition, the sectionalizing valve before and after the sectionalizing is called a "sectionalizing valve" without distinction in some cases.

The sectionalizing valve includes, for example, a split butterfly valve, a two-disc sectionalizing valve, a two-leaf split butterfly valve, a split valve, and the like.

In addition, the sectionalizing valve may be fixed to the container or may be detachably fixed to the container.

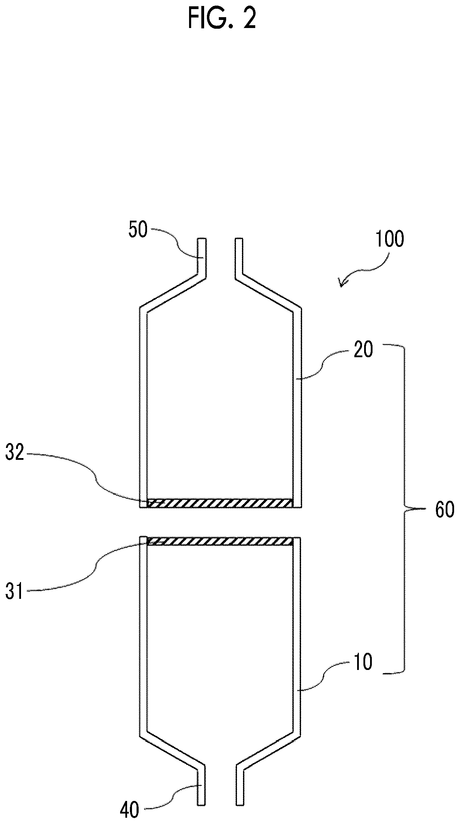

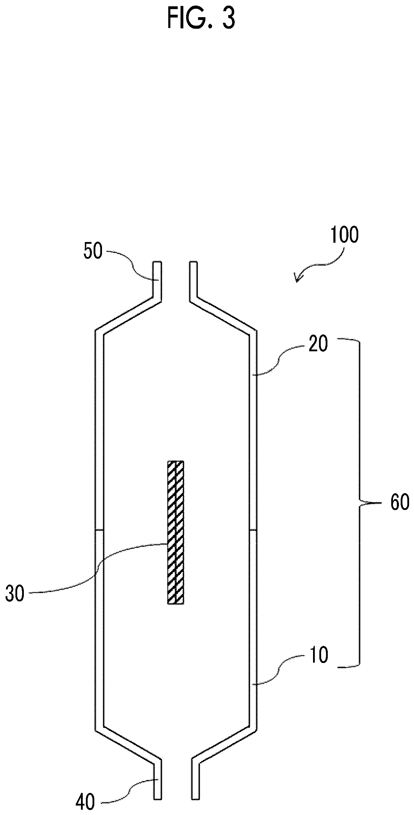

For example, in the heating treatment apparatus 100 shown in Fig. 1, the first chamber 10 and the second chamber 20 can be separated from each other in a state of being sealed with the sectionalizing valve 30. The sectionalizing valve 30 has a sectionalizing valve 31 on a first chamber side and a sectionalizing valve 32 on a second chamber side as shown in Fig. 2 and can perform an opening and closing operation as an integrated valve as shown in Fig. 3. The first chamber 10 after separation is sealed with the sectionalizing valve 31 after sectionalizing, while the second chamber 20 after separation is sealed with the sectionalizing valve 32 after sectionalizing.

In addition, the first chamber 10 and the second chamber 20 can be connected to each other via the sectionalizing valve 30. In this case, the sectionalizing valve 31 and the sectionalizing valve 32 after sectionalizing are connected to each other, but vacuum exhaust treatment may be performed between the sectionalizing valve 31 and the sectionalizing valve 32 from a perspective of more easily suppressing mixing with foreign substances.

For example, the heating treatment apparatus 100 shown in Fig. 1 is provided in the direction in which the aeration direction is along the vertical direction such that the air supply port 40 is on a lower side and the air exhaust port 50 is on an upper side. The first chamber 10 accommodates a heating treatment target object. In a state where the sectionalizing valve 30 is opened as shown in Fig. 3, a heated gas is flowed into the container 60 via the air supply port 40, and the heated gas is flowed out from the inside of the container 60 via the air exhaust port 50. Accordingly, the heated gas can be flowed into the container 60, and heating treatment can be performed on the heating treatment target object. In this case, since the heating treatment target object is heated with the heated gas and for example, moisture in the heating treatment target object is deprived and discharged, the heating treatment target object is dried. In addition, for example, a material configuring the heating treatment target object is crosslinked, and thereby a crosslinked substance of the heating treatment target object can be obtained.

After then, after the first chamber 10 and the second chamber 20 are separated from each other and the first chamber 10 sealed with the sectionalizing valve 31 is connected to an external space via the sectionalizing valve 31, a heating treatment object can be taken out to the external space from the first chamber 10 by opening the sectionalizing valve 31.

In addition, for example, the heating treatment apparatus 100 shown in Fig. 1 is provided in the direction in which the aeration direction is along the vertical direction such that the air exhaust port 50 is on the lower side and the air supply port 40 is on the upper side. The second chamber 20 accommodates a heating treatment target object. In a state where the sectionalizing valve 30 is opened, a heated gas is flowed into the container 60 via the air supply port 40, and the heated gas is flowed out from the inside of the container 60 via the air exhaust port 50. In the embodiment, as a distance between the air supply port 40 and the heating treatment target object increases, contact between the heated gas and the heating treatment target object becomes gentle. For this reason, in a case where flowing, scattering, or the like of the heating treatment target object caused by the heated gas becomes a problem, such an embodiment is preferable.

In addition, for example, the heating treatment apparatus 100 shown in Fig. 1 is provided in a direction in which the aeration direction is perpendicular to the vertical direction. Both of the first chamber 10 and the second chamber 20 accommodate a heating treatment target object. In a state where the sectionalizing valve 30 is opened as shown in Fig. 3, a heated gas may be flowed into the container 60 via the air supply port 40, and the heated gas may be flowed out from the inside of the container 60 via the air exhaust port 50. Accordingly, the heated gas can be flowed into the container 60, and heating treatment can be performed on the heating treatment target object. After then, after the first chamber 10 and the second chamber 20 are separated from each other and the first chamber 10 sealed with the sectionalizing valve 31 and the second chamber 20 sealed with the sectionalizing valve 32 are connected to an external space via the sectionalizing valve 31 and the sectionalizing valve 32 respectively, a heating treatment object can be taken out to the external space from the first chamber 10 and the second chamber 20 by opening the sectionalizing valve 31 and the sectionalizing valve 32.

For example, as the first chamber 10 and the second chamber 20 accommodate identical heating treatment target objects, many identical heating treatment objects can be manufactured. In addition, as the first chamber 10 and the second chamber 20 accommodate different heating treatment target objects, different heating treatment objects can be collectively manufactured.

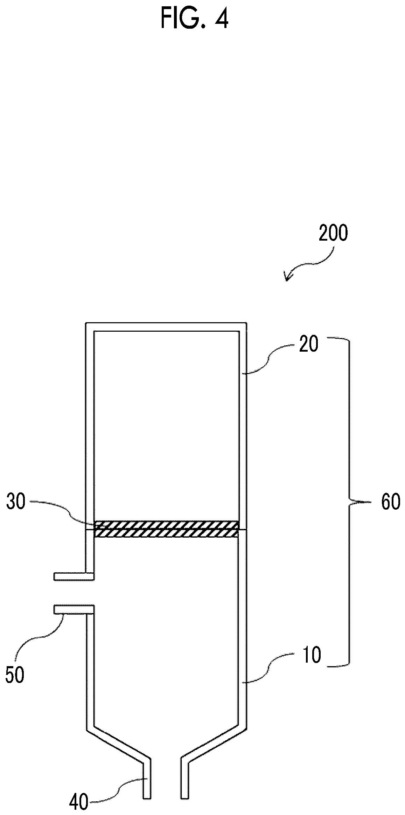

For example, in the container 60 of a heating treatment apparatus 200 shown in Fig. 4, the first chamber 10 are provided with both of the air supply port 40 and the air exhaust port 50.

For example, the heating treatment apparatus 200 is provided in the direction in which the aeration direction (the longitudinal direction of the container) is along the vertical direction such that the air supply port 40 is on the lower side. After the second chamber 20 that accommodates a heating treatment target object and the empty first chamber are connected to each other, the sectionalizing valve 30 is opened, and a part of the heating treatment target object accommodated in the second chamber 20 is accommodated in the first chamber, the sectionalizing valve 30 is closed. In a state where the sectionalizing valve 30 is closed, a heated gas is flowed into the container 60 via the air supply port 40, and the heated gas is flowed out from the inside of the container 60 via the air exhaust port 50. Accordingly, the heated gas can be flowed into the container 60, and heating treatment can be performed on the heating treatment target object. After then, after the first chamber 10 and the second chamber 20 are separated from each other and the first chamber 10 sealed with the sectionalizing valve 31 is connected to the outside via the sectionalizing valve 31, a heating treatment object can be taken out to the outside from the first chamber 10 by opening the sectionalizing valve 31.

Further, by again connecting the first chamber 10 after the heating treatment object is taken out to the second chamber 20, the heating treatment object can be obtained in the same manner above. Such heating treatment is effective in a case of manufacturing the heating treatment object little by little. In addition, by accommodating many heating treatment target objects in the second chamber 20 in advance, the heating treatment target objects can be held in a state of being sealed with the sectionalizing valve 32. Thus, mixing of foreign substances with the heating treatment target objects can also be easily suppressed compared to a case where the heating treatment target objects are accommodated in the first chamber 10 from the outside at each time of heating treatment.

As the heating treatment apparatus is sealed with the sectionalizing valve after heating treatment by a flow of a heated gas, a positive pressure in the container is easily maintained. For this reason, for example, maintaining of a dried state, an aseptic state, and the like is effective.

Since the heating treatment apparatus according to the embodiment of the present disclosure can heat a heating treatment target object in a static state and conditions such as the temperature of a heated gas can be adjusted, generation of static electricity of the heating treatment target object can be suppressed, for example, compared to an apparatus in which a fluidized drying type or vibration drying type container operates. For this reason, the heating treatment apparatus according to the embodiment of the present disclosure can be suitably used in performing heating treatment on the heating treatment target object such as powder and granules in which static electricity is likely to be generated. In addition, in a case where the heating treatment target object is porous, since removal of static electricity of pores is difficult, the heating treatment apparatus is effective in suppressing generation of static electricity.

In addition, since the heating treatment apparatus according to the embodiment of the present disclosure can use the heating treatment target object in a static state, coagulation of the heating treatment target object is suppressed, making coarsening unlikely, and unnecessary miniaturization can be suppressed, compared to an apparatus in which a fluidized drying type or vibration drying type container operates. Thus, a heating treatment object having a desired size is easily obtained.

[Temperature Adjusting Member]

The heating treatment apparatus may comprise a temperature adjusting member that is disposed on an outer side of the container and that is detachably attached to the heating treatment apparatus. Since a temperature inside the container can be adjusted via the container by using the temperature adjusting member, uniformly heating a heating treatment target object becomes even easier. In addition, by removing the temperature adjusting member from the heating treatment apparatus after heating treatment, handling of the heating treatment apparatus becomes even easier.

The temperature adjusting member is not particularly limited insofar as a temperature inside the container can be adjusted via the outer side of the container.

The temperature adjusting member may be in contact with the outer side of the container or may be contactless with the outer side of the container. The temperature adjusting member may be disposed to cover the outer side of the container or in the vicinity of the outer side of the container.

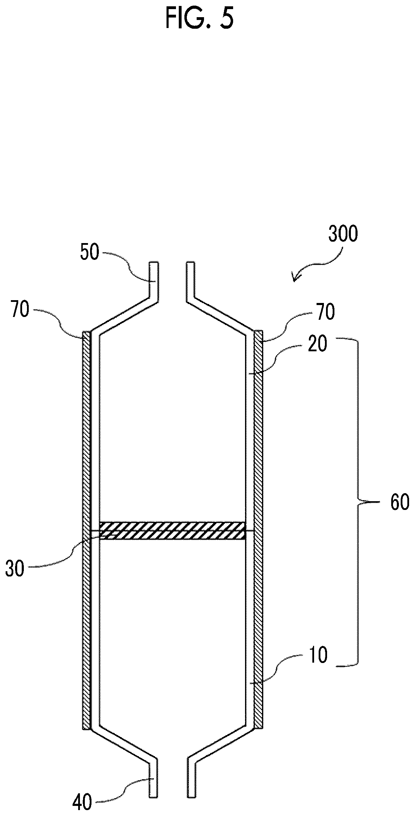

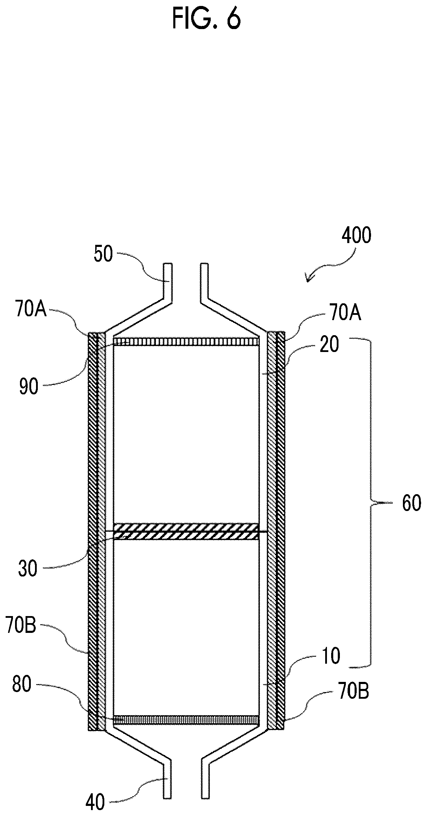

In addition, the temperature adjusting member may be disposed at a part of the outer side of the container or may be disposed over the entire periphery of the outer side of the container. For example, as in heating treatment apparatuses 300 and 400 shown in Figs. 5 and 6 respectively, temperature adjusting members 70, 70A, and 70B may be disposed on an outer side of the container 60 in the aeration direction.

The temperature adjusting member may be, for example, a heater that comprises a heating wire or may be, for example, a jacket type heater or the like. Accordingly, since a heated gas can be flowed into the container while a temperature inside the container is increased via the outer side of the container, uniformly heating a heating treatment target object becomes even easier. In the jacket type heater, a pitch of the heating wire is changed to obtain a desired heating wire pattern, and changing (for example, making thinner) the thickness of a jacket is easy.

The temperature adjusting member may be, for example, a metal member formed of a metal plate or the like. Since the metal member has high thermal conductivity compared to air and temperature unevenness of the outer side of the container reduces, making a temperature inside the container more uniform is easy, and uniformly heating a heating treatment target object becomes even easier.

From a perspective of reducing temperature unevenness, in a case where the temperature adjusting member is a metal member, the metal member is preferably formed of a metal plate having a certain thickness (for example, exceeding 20 mm). In a case where the metal member is formed of the metal plate, the thickness may be adjusted as appropriate depending on the type of the metal or the like, and shape processing may be performed as appropriate to be suitable for the shape of the container.

In addition, in a case where the temperature adjusting member is the metal member, in order to impart heat generating properties, the metal member may comprise a heater on an inner side or an outer side thereof. For example, the metal member may be connected to an external heat source or may be a cast heater. In a case where the metal member is the cast heater, the thickness of the cast heater is preferably larger than a pitch of a heater wire.

In a case where the temperature adjusting member is a metal member, a material is not particularly limited, but the temperature adjusting member is preferably made of stainless steel or made of aluminum.

Since stainless steel has a large heat capacity, a change in the temperature is small, and thermal conductivity is likely to be uniform. In addition, since aluminum has high thermal conductivity, fast heating is possible.

There may be one temperature adjusting member or may be two or more temperature adjusting members.

The temperature adjusting member may include at least one of the jacket type heater or the metal member.

For example, as in the heating treatment apparatus 400 shown in Fig. 6, the temperature adjusting member 70A that is a metal plate which covers the outer side of the container 60 may be disposed to cover the outer side of the container 60, and the temperature adjusting member 70B that is a jacket type heater may cover the temperature adjusting member 70A. Accordingly, since a temperature inside the container can be more uniformly increased via the outer side of the container while reducing the temperature unevenness of the outer side of the container, uniformly heating a heating treatment target object becomes even easier.

[Holding Member and Rectifying Member]

The heating treatment apparatus may include a holding member that holds a heating treatment target object between the air supply port and the air exhaust port. Accordingly, holding the heating treatment target object in the container becomes easier.

A material, a shape, or the like of the holding member is not particularly limited and may be selected as appropriate depending on the type of a heating treatment target object or the like. For example, the holding member is made of ceramics, is made of a metal (for example, SUS304 or the like), or the like. From a perspective of more uniformly heating the heating treatment target object, the holding member preferably comprises a ventilation hole and has a ventilation property. For example, the holding member may be a porous plate, a mesh plate, a punching metal, or a breathable bag.

In a case where the holding member comprises the ventilation hole, an opening ratio is not particularly limited, but may be approximately 5% to 30%.

In a case where the holding member has a ventilation property, the holding member can function as the rectifying member. That is, as a heated gas flowing in the container passes through the holding member via the ventilation hole of the holding member, a flow of the heated gas can be organized.

In a case where the holding member functions as the rectifying member, the holding member preferably has a plate shape. Accordingly, since the flow (resistance) of the heated gas flowing in the container can be sufficiently received and the linear speed of the heated gas can be made constant from a central portion to an edge portion of the holding member, the heating treatment target object can be more uniformly heated. From a perspective of further increasing a rectifying effect, the holding member preferably has a certain thickness (for example, 1/2 times to 3 times the diameter of the ventilation hole).

The container may have the rectifying member that has the same configuration as the holding member having a ventilation property in addition to the holding member. In a case where the holding member has a ventilation property, a heating treatment target object can be more uniformly heated by also serving as the rectifying member.

The position and number of holding members and the position and number of rectifying members are not particularly limited and may be selected as appropriate depending on design of the heating treatment apparatus or the like. For example, as in the heating treatment apparatus 400 shown in Fig. 6, a holding member 80 (serving as the rectifying member) may be provided in the vicinity of the air supply port 40 of the first chamber 10, and a rectifying member 90 may be provided in the vicinity of the air exhaust port 50 of the second chamber 20. The heating treatment apparatus shown in Fig. 6 can perform control such that a heated gas flows in a direction perpendicular to a holding member surface (holding surface).

In addition, as the container is provided in the direction in which the aeration direction (the longitudinal direction of the container) is along the vertical direction, a flow of a heated gas flowing in the container becomes more uniform. For this reason, uniformly heating a heating treatment target object becomes even easier.

Further, as the container is provided in the machine direction (that is, the direction in which the aeration direction is along the vertical direction), the filling proportion of the heating treatment target object can be further increased, and for example, achieving the filling proportion of 70% to 90% (most preferably 100%) becomes easier.

[Heated Gas]

A gas is not particularly limited, but is preferably an inert gas such as nitrogen and argon. In addition, a gas having a moisture content of 0.000613 g/m$^3$ (dew point: -80°C) to 4.85 g/m$^3$ (dew point: 0°C) is preferably used. A heated gas may be obtained by heating a gas through a known method.

[Heating Treatment Target Object]

A heating treatment target object is not particularly limited and may be a solid. Examples of a solid heating treatment target object include powder, granules, and the like.

The heating treatment apparatus according to the embodiment of the present disclosure can be suitably used for manufacturing a polymer porous crosslinked substance by performing heating treatment on a bioaffinity polymer such as collagen and gelatin or a polymer porous substance thereof.

[Others]

The heating treatment apparatus may include a valve for controlling a flow of a heated gas. For example, the valve may be disposed in the vicinity of the air supply port, in the vicinity of the air exhaust port, or the like.

In addition, the heating treatment apparatus may include an aseptic filter. For example, the aseptic filter may be disposed in the vicinity of the air supply port, in the vicinity of the air exhaust port, or the like.

In order to flow heated air to the container, the heating treatment apparatus may include a pump or an air supply fan and may be connected to an external pump or the air supply fan. In addition, an air supply source may be a compressor or may be a compression cylinder or the like.

<Manufacturing Method of Heating Treatment Object>

The manufacturing method of a heating treatment object according to the embodiment of the present disclosure includes a step of accommodating a heating treatment target object in the container of the heating treatment apparatus according to the embodiment of the present disclosure (hereinafter, called an "accommodating step" in some cases) and a step of flowing a heated gas in the container via the air supply port and the air exhaust port (hereinafter, called a "heated gas flowing step" in some cases).

Accordingly, heat treatment can be performed on the heating treatment target object, and thereby a heating treatment object can be manufactured.

The manufacturing method of a heating treatment object according to the embodiment of the present disclosure may include a step of collecting a heating treatment object by closing the sectionalizing valve (hereinafter, called a "collecting step" in some cases) after the heated gas flowing step. Accordingly, the heating treatment object can be taken out while suppressing mixing with foreign substances.

A heating treatment target object and a heated gas are as described above.

[Accommodating Step]

In the accommodating step, a method of accommodating a heating treatment target object in the container is not particularly limited. For example, the heating treatment target object may be accommodated in the container by opening the sectionalizing valve or removing the sectionalizing valve from the container. In addition, for example, the heating treatment target object may be accommodated in the container via the supply port provided in the container.

[Heated Gas Flowing Step]

The temperature of a heated gas and a treatment time (that is, a time for which the heated gas flows) are not particularly limited and may be selected as appropriate in consideration of an object or the like of heating treatment.

An inflow speed and an inflow amount of the heated gas are not particularly limited and may be selected as appropriate depending on properties of a heating treatment target object, an object of heating treatment, or the like. For example, the heating treatment target object may be flowed by increasing at least one of the inflow speed or the inflow amount of the gas.

A polymer porous substance is placed in the container of the heating treatment apparatus, and a heated gas is flowed via the air supply port and the air exhaust port. Accordingly, heat treatment can be performed on a polymer porous substance (heating treatment target object) to crosslink the polymer porous substance, and thereby a polymer porous crosslinked substance (heating treatment object) can be manufactured.

A crosslinking temperature (that is, the temperature of a heated gas) is preferably 100°C to 200°C, more preferably 120°C to 170°C, and even more preferably 130°C to 160°C. By adopting a thermal crosslinking method, the use of a crosslinking agent can be avoided. The treatment time of the thermal crosslinking varies depending on a crosslinking temperature, the type of a polymer, or a degree of degradation to be maintained.

In order to flow heated air in the container, the pump provided in the heating treatment apparatus or the external pump connected to the heating treatment apparatus may be used.

The manufacturing method of a heating treatment object may include a step in which the heating treatment apparatus comprises the temperature adjusting member and the container is heated by the temperature adjusting member. Accordingly, since a temperature inside the container can be adjusted, uniformly heating a heating treatment target object becomes even easier.

In the manufacturing method of a heating treatment object, the temperature adjusting member may include stainless steel and a heater that heats stainless steel. In addition, at least a part of the step of flowing a heated gas and at least a part of the step of heating the container may be simultaneously performed. Accordingly, since a temperature inside the container can be more uniformly increased via the outer side of the container while reducing the temperature unevenness of the outer side of the container, uniformly heating a heating treatment target object becomes even easier. In addition, since the temperature inside the container can be adjusted via the outer side of the container by using the temperature adjusting member, uniformly heating the heating treatment target object becomes even easier.

From a perspective of more uniformly heating the heating treatment target object, it is preferable that the step of flowing the heated gas and the step of heating the container are simultaneously performed.

Hereinafter, a case where heating treatment is performed on a polymer porous substance (heating treatment target object) of a bioaffinity polymer such as gelatin and collagen and thereby a polymer porous crosslinked substance (heating treatment object) is manufactured will be given as an example, and the manufacturing method of a heating treatment object according to the embodiment of the present disclosure will be further described.

-Polymer Porous Substance-

A polymer porous substance (heating treatment target object) is preferably a porous block that consists of a polymer.

As in the manufacturing method to be described later, after a polymer aqueous solution containing a polymer is placed into a liquid container to perform a freezing step and a polymer aqueous solution frozen substance obtained by freezing the polymer aqueous solution is obtained, the polymer porous substance is obtained by performing a moisture removing step on the polymer aqueous solution frozen substance and removing moisture.

A pulverizing step, a classification step, and the like to be described later may be further performed on the polymer porous substance. Accordingly, the polymer porous substance can be processed into granules having various sizes. In addition, a filling step or the like to be described later may be further performed on the polymer porous substance.

For example, a thermal crosslinking condition in a case where a recombinant peptide CBE3 is used as human-derived recombinant gelatin is as follows. In a case where the actual temperature is approximately 135°C, the treatment time is preferably 2 hours to 20 hours, more preferably 3 hours to 18 hours, and even more preferably 4 hours to 8 hours. From a perspective of oxidation prevention, treatment of thermal crosslinking is preferably performed in an atmosphere of an inert gas (that is, a heated gas). For example, it is preferable to perform thermal crosslinking for 3 hours to 7 hours in a nitrogen atmosphere at 130°C to 150°C. Nitrogen or argon is preferable as the inert gas, and from a perspective of uniform heating, crosslinking in an inert gas atmosphere is more preferable than crosslinking under a vacuum.

-Polymer Porous Crosslinked Substance-

The polymer porous crosslinked substance obtained as described above is suitable as a cell scaffold, a transplantation member, a tissue repair material, or the like.

(Acid Residual Rate)

A polymer porous crosslinked substance preferably indicates a predetermined acid residual rate. For example, the acid residual rate obtained through 3 hours of decomposition treatment using 1 mol/L hydrochloric acid is preferably 30% to 70%, more preferably 35% to 65%, and even more preferably 45% to 65% by mass.

The "acid residual rate" of the polymer porous crosslinked substance is a physical property value measured as follows. The mass of a measuring microtube (trade name: Mini Supertube, manufactured by IBIS Corporation, capacity: 2 ml, hereinafter referred to as a tube) is measured (A). For a granular polymer porous crosslinked substance, 15.0 ($\pm$0.2) mg is weighed (n = 3) without processing, and for a block-shaped polymer porous crosslinked substance, a cylindrical sample having diameter 6 mm $\times$ thickness approximately 1 mm is produced, the mass is measured (B), and the sample fills the measuring tube. 1.7 ml of 1 mol/L HCl is added to the tube in which the polymer porous crosslinked substance is placed, and the mixture is allowed to stand at a constant temperature of 37 $\pm$0.5°C for 3 hours. After a specified time, the tube is placed on ice to stop reaction, and the mixture is centrifuged at 10,000$\times$g for 1 minute in a centrifuge previously set at 4°C. After confirming that a tissue repair material has settled, a supernatant is absorbed, ultrapure water previously cooled on ice is added, and centrifugation is performed again under the same conditions as described above. Absorbing the supernatant, again adding the ultrapure water, and again performing centrifugation under the same conditions as described above are repeated two times thereafter. After absorbing the supernatant, freeze-drying is performed. After taking out from a freeze-dryer, a tube cap is quickly closed in order to prevent the polymer porous crosslinked substance from absorbing moisture in the air. The mass of each tube is measured (C), and the acid residual rate is calculated using the following formula.

$$\text{Acid residual rate} = (C - A)/B \times 100(\%)$$

The acid residual rate of a polymer porous crosslinked substance differs depending on a component contained in the polymer porous crosslinked substance and particularly the type and form of granules, but can be adjusted by, for example, a temperature, a treatment time, or the like of a crosslinking step (that is, the heated gas flowing step). The acid residual rate tends to decrease as the treatment time shortens.

A polymer porous crosslinked substance may be stored in the container. For example, in a case of a polymer porous crosslinked substance that consists of gelatin, the container is not particularly limited, and for example, a glass vial hermetically sealed with a rubber stopper and an aluminum cap can be used. The size of the glass vial is not particularly limited. The glass vial may be subjected to silicone resin coating using dimethylpolysiloxane or the like, fluororesin coating, silica coating, dealkalising treatment, or the like. The various types of coating or dealkalising treatment can provide as antistatic, antiadhesive, and water repellent effects and the like. In addition, the container may be further packaged. The packaging is also not particularly limited, and for example, an aluminum pouch can be used.

Hereinafter, a manufacturing method of a polymer porous substance (heating treatment target object) will be described.

[Manufacturing Example of Polymer Porous Substance]

(Polymer)

In the present disclosure, the term "polymer" refers to a molecule having a large molecular weight and a molecule having a structure configured by a number of repetitions of a unit obtained substantially or conceptually from a molecule having a small molecular weight. Examples thereof include polyamine, cellulose, amylose, starch, chitin, a polypeptide, protein, DNA, RNA, and the like. The polymer is preferably water-soluble and more preferably a polypeptide or protein. Among a polypeptide and protein, collagen and gelatin are particularly preferable.

A hydrophilic repeating unit ratio in a polymer is preferably 50% or less and more preferably 30% or less. In a case where a hydrophilic unit ratio is higher than the hydrophilic repeating unit ratio, the amount of free water around the polymer is reduced, and freezing is inhibited. Herein, the hydrophilic repeating unit ratio refers to the ratio of repeating units having an ionic group and/or a hydroxyl group in the polymer.

The gelatin means a polypeptide containing six or more consecutive sequences represented by Gly-X-Y and may have one or more other amino acid residues in a polypeptide in addition to a sequence represented by Gly-X-Y in the polypeptide. The sequence represented by Gly-X-Y is a sequence corresponding to an amino acid sequence derived from a partial amino acid sequence of collagen, and the repetition of the sequence means a sequence characteristic of collagen.

A plurality of pieces of Gly-X-Y may be the same or may be different from each other. In addition, X and Y in a Gly-X-Y sequence are independent of each other for each repeating unit and may be the same or different from each other. In Gly-X-Y, Gly represents a glycine residue, and X and Y represent any amino acid residue other than the glycine residue. It is preferable that a large amount of imino acid residues, that is, proline residues or oxyproline residues are contained as X and Y The content of such imino acid residues preferably occupies 10% to 45% of the entire gelatin. The content of Gly-X-Y in the gelatin is preferably 80% or more, more preferably 95% or more, and most preferably 99% or more.

The gelatin may be a natural type or may be a mutant type in which at least one amino acid residue is different from the natural type. The natural type gelatin means a polypeptide that has gelatin having naturally occurring collagen as a raw material or the same amino acid sequence as the gelatin having naturally occurring collagen as a raw material. Unless stated otherwise, in the present disclosure, mutant type gelatin or gelatin of recombinant are collectively referred to as recombinant gelatins. Examples of the natural type gelatin or recombinant gelatin thereof include those derived from an animal such as a fish and a mammal, but natural type gelatin or recombinant gelatin thereof of a mammalian animal is preferable. Examples of the mammalian animal include humans, horses, pigs, mice, rats, and the like, and humans or pigs are more preferable. The natural type gelatin is preferably derived from pigs or humans, and the recombinant gelatin is preferably human-derived recombinant gelatin.

In addition, the gelatin is preferably recombinant gelatin obtained by introducing, into a proper host, a base sequence or an amino acid sequence in which one or more bases or amino acid residues are modified with respect to the base sequence or the amino acid sequence of a gene encoding the collagen having six or more consecutive sequences represented by the Gly-X-Y, through a conventional method. By using such recombinant gelatin, there are advantages such as a (bone) tissue repairing ability can be enhanced, various properties can be exhibited compared to a case where natural gelatin is used, and effects of inconveniences such as rejection reaction caused by a living body can be avoided.

The recombinant gelatin disclosed in, for example, EP1014176B, US6992172B, WO2004/85473A, WO2008/103041A, JP2010-519293A, JP2010-519252A, JP2010-518833A, JP2010-519251A, WO2010/128672A, WO2010/147109A, and the like can be particularly preferably used. In addition, the recombinant gelatin has a molecular weight that is preferably 2 kDa or more and 100 kDa or less, more preferably 5 kDa or more and 90 kDa or less, and even more preferably 10 kDa or more and 90 kDa or less.

From a perspective of bioaffinity, the recombinant gelatin preferably further includes a cell adhesion signal and more preferably has two or more cell adhesion signals, which are present in the recombinant gelatin, in one molecule. Examples of such a cell adhesion signal include an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and a HAV sequence, the examples preferably include the RGD sequence, the YIGSR sequence, the PDSGR sequence, the LGTIPG sequence, the IKVAV sequence, and the HAV sequence, and the examples particularly preferably include the RGD sequence. Among the RGD sequences, an ERGD sequence is more preferable.

Regarding the disposition of the RGD sequences in the recombinant gelatin, the number of amino acid residues between the RGDs is preferably 0 to 100 and more preferably 25 to 60. In addition, it is preferable that the RGD sequences are non-uniformly disposed within a range of the number of such amino acid residues. In addition, the ratio of the RGD sequences to the total number of amino acid residues in the recombinant gelatin is preferably at least 0.4%, and in a case where the recombinant gelatin contains 350 or more amino acid residues, each stretch of the 350 amino acid residues preferably contains at least one RGD sequence.

The recombinant gelatin preferably contains at least two RGD sequences per 250 amino acid residues, more preferably contains at least three RGD sequences, and even more preferably contains at least four RGD sequences. However, the sequence of the recombinant gelatin preferably has the following aspects: (1) does not contain a serine residue and a threonine residue, (2) does not contain a serine residue, a threonine residue, an asparagine residue, a tyrosine residue, and a cysteine residue, and (3) does not contain an amino acid sequence represented by Asp-Arg-Gly-Asp. The recombinant gelatin may have one of aspects (1) to (3) of the preferable sequence or may have a combination of two or more aspects. In addition, the recombinant gelatin may be partially hydrolyzed.

The recombinant gelatin preferably has a repeating structure of A-[(Gly-X-Y)n]m-B. m represents 2 to 10 and preferably represents 3 to 5. A and B represent any amino acid or any amino acid sequence. n represents 3 to 100, preferably represents 15 to 70, and more preferably represents 50 to 60.

The recombinant gelatin is preferably represented by formula Gly-Ala-Pro-[(Gly-X-Y)63]3-Gly (in the formula, 63 Xs each independently represent any amino acid residue, 63 Ys each independently represents any amino acid residue, and three pieces of (Gly-X-Y)63 may be the same or different from each other).

A plurality of naturally occurring collagen sequence units are preferably bonded to the repeating unit of the recombinant gelatin. Examples of the naturally occurring collagen referred to herein preferably include type I, type II, type III, type IV, and type V. Examples thereof more preferably include type I, type II, or type III. Examples of the origin of the collagen include humans, horses, pigs, mice, and rats, and humans are more preferable.

An isoelectric point of the recombinant gelatin is preferably 5 to 10, more preferably 6 to 10, and even more preferably 7 to 9.5.

Preferred aspects of the recombinant gelatin include: a substantially pure collagen material (1) in which a carbamoyl group is not hydrolyzed, (2) not having procollagen, (3) not having telopeptide, and (4) produced from nucleic acid encoding natural collagen. The recombinant gelatin may have one of the preferred aspects (1) to (4) or may have a combination of two or more aspects.

The recombinant gelatin can be preferably any one of the following (A) to (C) from the height of the (bone) tissue repairing ability.

(A) A polypeptide represented by the following sequence number 1,

GAP

(GAPGLQGAPGLQGMPGERGAAGLPGPKGERGDAGPKGADGAPGAPGLQGMPGERG

AAGLPGPKGERGDAGPKGADGAPGKDGVRGLAGPIGPPGERGAAGLPGPKGERGDA

GPKGADGAPGKDGVRGLAGPIGPPGPAGAPGAPGLQGMPGERGAAGLPGPKGERGD

AGPKGADGAPGKDGVRGLAGPP)3G (sequence number 1)

(B) in the amino acid sequence of (A), a polypeptide that has a partial amino acid sequence consisting of 4th to 192nd amino acid residues and a partial sequence having 80% or more sequence identity and that has the (bone) tissue repairing ability, and

(C) a polypeptide that consists of an amino acid sequence in which one or several amino acid residues are deleted, substituted, or added to the amino acid sequence of (A) and that has the (bone) tissue repairing ability.

From a perspective of the (bone) tissue repairing ability of the recombinant gelatin, the sequence identity in (B) is more preferably 90% or more and even more preferably 95% or more.

The partial amino acid sequence in the sequence of (B) is a partial amino acid sequence corresponding to the repeating unit of the sequence represented by sequence number 1. In a case where a plurality of partial amino acid sequences corresponding to the repeating unit are present in the polypeptide of (B), a polypeptide can contain one repeating unit and preferably two or more repeating units having 80% or more sequence identity.

In addition, the polypeptide specified in (B) preferably contains, as the total number of amino acid residues, 80% or more of the number of all amino acid residues of partial sequences having 80% or more sequence identity with the partial amino acid sequence corresponding to the repeating unit.

As the length of the polypeptide specified in (B), the number of amino acid residues can be 151 to 2,260, the number of amino acid residues is 193 or more from a perspective of degradability after crosslinking and is preferably 944 or less from a perspective of stability, and the number of amino acid residues is more preferably 380 to 756.

In addition, the polypeptide specified in (C) may be a polypeptide that consists of an amino acid sequence in which one or several amino acid residues are deleted, substituted, or added to the amino acid sequence of (A) and that has the tissue repairing ability.

The number of amino acid residues deleted, substituted, or added in the polypeptide specified in (C) may be one or more and varies depending on the total number of amino acid residues of the recombinant gelatin, but can be, for example, 2 to 15 and preferably 2 to 5.

The recombinant gelatin can be manufactured through gene recombination technology which is known to those skilled in the art and can be manufactured in accordance with methods disclosed in, for example, EP1014176B, US6992172B, WO2004/85473A, WO2008/103041A, and the like. Specifically, a gene encoding an amino acid sequence of predetermined recombinant gelatin is obtained, the gene is incorporated into an expression vector to produce a recombinant expression vector, and the expression vector is introduced into a proper host to produce a transformant. Since the recombinant gelatin is produced by culturing the obtained transformant in a suitable culture medium, the recombinant gelatin used in the present disclosure can be prepared by collecting the recombinant gelatin produced from the culture.

(Polymer Aqueous Solution)

A polymer aqueous solution contains one or more types of polymers. The polymer concentration of the polymer aqueous solution is preferably 0.1% by mass or more, more preferably 1% by mass or more, and even more preferably 5% by mass or more. In a case where the concentration is lower than 0.1% by mass, it is difficult to maintain the structure of a polymer porous substance after removing moisture. The polymer aqueous solution is preferably gelled at a freezing temperature or higher. An upper limit of the polymer concentration in the polymer aqueous solution is not particularly limited insofar as the polymer can be dissolved, but is generally 40% by mass or less or may be 30% by mass or less or 20% by mass or less.

The polymer aqueous solution is produced by purifying and concentrating a solution containing the polymer or by dissolving the polymer in a dried state in an aqueous medium. (1) Coordination of errands may be performed or (2) a pre-adjusted product may be prepared and used. (3) The polymer aqueous solution may be adjusted by freeze-drying the polymer aqueous solution obtained by purification and concentration and adding an aqueous medium to the obtained freeze-dried substance to be redissolved. Alternatively, (4) the polymer aqueous solution may be adjusted by freezing the polymer aqueous solution obtained by purification and concentration and thawing the obtained frozen substance. The frozen substance is preferably thawed at 30°C to 40°C for 15 to 20 hours from a perspective of reducing generation of air bubbles and insoluble matters (unmelted residues of the frozen substance). The method (4) is preferable from a perspective of reduction of the time and effort required for coordination of errands, convenience of transportation and storage, and reduction of air bubbles and insoluble matters in the polymer aqueous solution.

It is preferable that air bubbles and insoluble matters dispersed in the polymer aqueous solution are removed before the freezing step through operations such as filtration, centrifugation, depressurization, and defoaming. As a result, the yield of the frozen polymer aqueous solution having low anisotropy improves. The removal of the air bubbles and the insoluble matters can be evaluated through turbidity measurement. Alternatively, the evaluation can be made through visual test using an optical microscope. For example, the number of air bubbles and unnecessary substances reflected in the visual field of the optical microscope can be calculated and evaluated based on the number of air bubbles and insoluble matters in 1 $\mu$L of the polymer aqueous solution. The air bubbles and the insoluble matters in the polymer aqueous solution are preferably 0.5 pieces/$\mu$L or less, more preferably 0.3 pieces/$\mu$L or less, even more preferably 0.1 pieces/$\mu$L or less, and particularly preferably 0 pieces/$\mu$L.

Components other than a polymer may be added to the polymer aqueous solution for the purpose of adding predetermined properties. Examples of such other components include a component related to bone regeneration or osteogenesis, such as an osteoinductive agent. Examples of the osteoinductive agent include bone morphogenetic protein (BMP) and a basic fibroblast growth factor (bFGF), and the osteoinductive agent is not particularly limited. In addition, examples thereof include a crosslinking agent of a polypeptide or protein.

The aqueous medium of the polymer aqueous solution is not particularly limited insofar as a medium can dissolve a polymer and can be used for a biological tissue. Examples thereof include a medium generally usable in the field of the art, such as water, physiological saline, and a phosphate buffer solution.

In a case where gelatin is used as a polymer, a gelatin concentration in a gelatin solution is not particularly limited insofar as the gelatin can be dissolved at the concentration. The gelatin concentration of the gelatin solution is, for example, preferably 0.5% by mass to 20% by mass, more preferably 2% by mass to 16% by mass, and even more preferably 4% by mass to 12% by mass. In addition, defoaming treatment may be performed on the gelatin solution before the freezing step. Accordingly, uniform formation of ice crystals can be made more likely to occur. A defoaming method is not particularly limited, but for example, vacuum centrifugal defoaming can be performed at a pressure of 2 to 10 kPa.

The gelatin solution may be filtered in order to remove undissolved particles. A filtration method is not particularly limited, but for example, pressurization filtration is performed using a filter having a pore diameter of 0.22 to 0.45 $\mu$m. A material for the filter is also not particularly limited, and polytetrafluoroethylene, polyether sulfone, cellulose acetate, polyvinylidene fluoride, or the like can be used. However, cellulose acetate is preferable from a perspective of low gelatin adsorption and few eluents. A temperature in a case of producing the gelatin solution is not particularly limited and may be a generally used temperature, for example, 0°C to 60°C and preferably approximately 3°C to 40°C.

(Liquid Container)

The liquid container is a container in which a polymer aqueous solution is placed and cooled/frozen. Examples of the shape of the container include a dish shape and a cylindrical cup shape. The cylindrical cup shape is preferable. It is preferable that the inside of the container does not have a high curvature. Specifically, the curvature is preferably R1 mm or more and more preferably R2 mm or more (R means a curvature radius). The size of the container is not particularly limited, but in a case of a cylindrical cup-shaped container, the inner diameter is preferably 200 mm or less and more preferably 150 mm or less.

An inner surface of the liquid container may be coated with the same or a different member (coating member) from a member configuring the liquid container. In addition, a cover member that consists of the same or the different member from the member configuring the liquid container may be spread on the inner surface of the liquid container or a cover member having a cylindrical shape may be provided. Distinguishing from the coating member and the cover member, the member configuring the liquid container will also be referred to as a major member of the liquid container. A combination of the major member of the liquid container, the coating member, and the cover member is not limited. That is, the liquid container may be any combination of (1) only the major member of the liquid container, (2) the major member of the liquid container and the coating member, (3) the major member of the liquid container and the cover member, and (4) the major member of the liquid container, the coating member, and the cover member.

The inner surface of the liquid container, that is, a surface in contact with the polymer aqueous solution in a case where the polymer aqueous solution is placed in the liquid container is made of a tetrafluoroethylene hexafluoropropylene copolymer (also referred to as perfluoroethylene propylene copolymer) (FEP). For example, according to (1) to (4), (1) the major member of the liquid container is made of FEP, (2) at least the coating member is made of FEP, (3) at least the cover member is made of FEP, and (4) at least the cover member is made of FEP.

A material for the major member of the liquid container is not particularly limited, and examples thereof include aluminum. In a case where the major member of the liquid container is in contact with the polymer aqueous solution, the major member of the liquid container is made of FEP. A material for the major member of the liquid container has linear expansion coefficiency (also referred to as thermal expansion coefficiency) that is preferably $10 \times 10^{-5}$/K or less, more preferably $50 \times 10^{-6}$/K or less, and particularly preferably $25 \times 10^{-6}$/K or less.

The cover member may be evenly spread on the container inner surface, and the shape and the thickness of the cover member are not limited. In a case where the cover member is in contact with the polymer aqueous solution, the cover member is made of FEP. The container inner surface may be evenly coated with the coating member, and the thickness of a coating film is not limited. The coating film is preferably 20 $\mu$m or more and more preferably 50 $\mu$m or more. In a case where the coating member is in contact with the polymer aqueous solution, the coating member is made of FEP.

(Freezing Step)

A step of freezing a polymer aqueous solution accommodated in the liquid container (hereinafter, called a "freezing step" in some cases) is performed. Accordingly, a polymer aqueous solution frozen substance can be obtained. A freezing unit is not particularly limited, and freezing may be performed by, for example, a device such as a refrigerator and a freeze-dryer. In a case of performing freezing by the freeze-dryer, it is possible for the same device to continuously remove moisture from the polymer aqueous solution frozen substance (freeze-drying).

The temperature of the freezing step varies depending on the type of a polymer and the concentration of the polymer aqueous solution, but a difference between the temperature of a portion having the highest liquid temperature in a solution immediately before the generation of solidification heat and the temperature of a portion having the lowest liquid temperature in the solution is preferably 2.5°C or lower. Herein, the "difference in temperature immediately before the generation of solidification heat" means a difference in temperature in a case where the difference in temperature becomes largest between 1 second and 10 seconds before the generation of solidification heat. In addition, the temperature of the portion having the lowest liquid temperature in the solution is preferably -15°C or higher and -8°C or lower and more preferably -15°C or higher and -10°C or lower.

In addition, by manufacturing the polymer aqueous solution frozen substance as described above, a probability in which a polymer aqueous solution frozen substance having low anisotropy is obtained can be improved.

The "anisotropy" of the polymer aqueous solution frozen substance means physical properties measured as follows. After freeze-drying the polymer aqueous solution frozen substance, a near central part of a freeze-dried substance (polymer porous substance) is cut in horizontal and perpendicular directions. Next, each cross section is dyed, and a fixed (2.0 mm × 2.0 mm or 2.5 mm × 2.5 mm) region is observed with the optical microscope. Among rectangles circumscribing a region surrounded by the dyed material in the part to be observed, a circumscribed rectangle in which a distance between two facing sides of the rectangle is maximum is selected. 50 lengths of long sides of the circumscribed rectangle in which the distance between the two facing sides is maximum are measured in the part to be observed in each of the cross section in the horizontal direction and the cross section in the perpendicular direction, and an average thereof is defined as an average value of major axes of a mesh of the frozen substance.

A ratio d2/d1 obtained with a smaller one of the major axis (average value) of the cross section in the horizontal direction and the major axis (average value) of the cross section in the perpendicular direction as d1 and the other one as d2 in each mesh in this case will be defined as "anisotropy". A polymer aqueous solution frozen substance having 3 or lower anisotropy will be defined as a polymer aqueous solution frozen substance having "low anisotropy".

From a perspective of manufacturing efficiency, the yield of the polymer aqueous solution frozen substance having low anisotropy is preferably 90% or more.

(Moisture Removing Step)

A step of removing moisture from a polymer aqueous solution frozen substance (hereinafter, called a "moisture removing step" in some cases) is performed. Accordingly, a polymer porous substance can be obtained. A unit that removes moisture is not particularly limited. There are a method of melting ice in the polymer aqueous solution frozen substance, a sublimating (freeze-drying) method, and the like, and freeze-drying is preferable. A freeze-drying period can be, for example, 0.5 hours to 300 hours. A freeze-dryer that can be used is not particularly limited.

(Pulverizing Step)

In the pulverizing step, a polymer porous substance is pulverized, thereby obtaining a pulverized material. For the pulverization, a pulverizer such as a hammer mill or a screen mill can be used. From a perspective of small variation in particle size distribution between tests, a screen mill (for example, Comil manufactured by Quadro Engineering) is preferable since this device makes it possible to collect materials pulverized in the same size as needed. As the condition of the pulverization, in order to maintain the structure of the surface of the pulverized material, a cutting method by crushing is preferable. In addition, in order to maintain the internal structure of granules, it is preferable to adopt a method in which the granules are not strongly compressed during pulverization.

(Classification Step)

For the purpose of sizing, a classification step can be included after the pulverizing step. Accordingly, a polymer pulverized material having a uniform particle diameter can be obtained. For example, in classifying pulverized gelatin materials, it is preferable to use a sieve having an opening size of 300 μm to 1,400 μm.

(Filling Step)

A step of filling a vial with a pulverized material can be included after the pulverizing step. For example, a filling method of a pulverized gelatin material is not particularly limited, but a mass feedback type table feeder can be used. A vial with which the pulverized gelatin material filled is also not particularly limited, but for example, a glass vial having a silicone-processed inner surface can be used.

Examples

Hereinafter, the present disclosure will be more specifically described with reference to Examples. However, the present disclosure is not limited to the Examples.

[Example]

(Heating Treatment Apparatus)

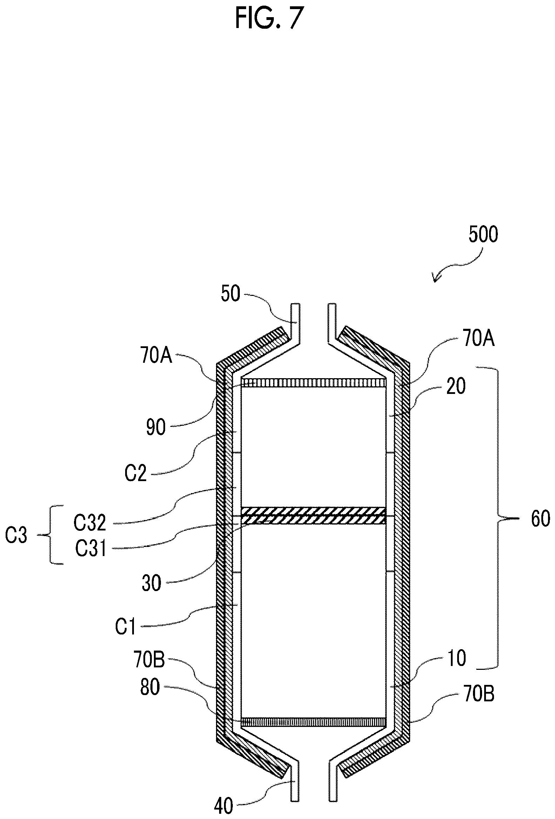

A heating treatment apparatus 500 shown in Fig. 7 is produced in the following manner.

-Preparation of Member-

· A first cylindrical member C1 having the air supply port 40
Made of SUS304, thickness: 3 mm, length of cylindrical portion in aeration direction: 20 cm, and inner diameter of cylindrical portion: 15 cm
· A second cylindrical member C2 having the air exhaust port 50
Made of SUS304, thickness: 3 mm, length of cylindrical portion in aeration direction: 10 cm, and inner diameter of cylindrical portion: 15 cm
· A third cylindrical member C3 provided with the sectionalizing valve 30
"PS150" manufactured by ChargePoint, and length in aeration direction: 15 cm

Along with sectionalizing of the sectionalizing valve 30, the third cylindrical member C3 is separated into a cylindrical member C31 (length in aeration direction: 7 cm) and a cylindrical member C32 (length in aeration direction: 8 cm) that are closed with the sectionalizing valve 30 after sectionalizing.

· Temperature adjusting member 70A
Made of SUS304 and thickness: 30 mm
· Temperature adjusting member 70B
Jacket type heater
· Holding member 80 and rectifying member 90
Punching metal made of SUS304, opening ratio: 10%, diameter of ventilation hole: 0.5 mm, and thickness: 0.2 mm

-Production-

The holding member 80 is disposed in the vicinity of the air supply port 40 in the first cylindrical member C1. A portion of the cylindrical member C31 of the third cylindrical member C3 provided with the sectionalizing valve 30 is connected to the cylindrical portion of the first cylindrical member C1. Accordingly, the first chamber 10 (the length of the cylindrical portion in the aeration direction: 27 cm) is formed.

The rectifying member 90 is disposed in the vicinity of the air exhaust port 50 in the second cylindrical member C2. A portion of the cylindrical member C32 of the third cylindrical member C3 is connected to the cylindrical portion of the second cylindrical member C2. Accordingly, the second chamber 20 (the length of the cylindrical portion in the aeration direction: 18 cm) is formed.

The temperature adjusting member 70A and the temperature adjusting member 70B are attached in a detachably state to the outer side of the container 60 including the first chamber 10 and the second chamber 20.

In this manner, the heating treatment apparatus 500 comprising the container 60 that includes the first chamber 10 and the second chamber 20 which are connected to each other via the sectionalizing valve 30 and the air supply port 40 and the air exhaust port 50 through which a heated gas flows is created. In a case of separating the first chamber 10 and the second chamber 20 that are connected to each other via the sectionalizing valve 30, the two chambers in a sealed state can be sectionalized into two respectively.

(Heating Treatment Target Object)

A polymer porous substance is prepared as a heating treatment target object in the following manner.

The following recombinant peptide CBE3 (described in WO2008/103041A) is prepared as recombinant gelatin.

CBE3 molecular weight: 51.6 kD, and structure: GAP[(GXY)63]3G
Number of amino acids: 571
RGD sequence: 12 sequences

Imino acid content: 33%

Almost 100% of amino acids have a repeating structure of GXY. In the amino acid sequence of CBE3, serine residues, threonine residues, asparagine residues, tyrosine residues, and cysteine residues are not contained. CBE3 has an ERGD sequence.

Isoelectric point: 9.34, and a hydrophilic repeating unit ratio in a polymer is 26.1%.

Amino acid sequence (sequence number 1)

A solution containing the recombinant gelatin is purified and then concentrated through ultrafiltration to 4.0% by mass at 30°C. After an obtained gelatin aqueous solution is freeze-dried, water for injection is added to a freeze-dried substance and is redissolved by increasing the temperature to 37°C for 30 minutes, and thereby a 7.5% by mass gelatin aqueous solution is obtained again. The gelatin aqueous solution is filtered through a 0.22 $\mu$m cellulose acetate film filter and is subjected to vacuum centrifugal defoaming at 4.0 kPa for 180 seconds using a vacuum defoaming machine (Kurabo, KK-V300SS-I). The gelatin aqueous solution is sampled in a transparent polystyrene container to have a liquid thickness of 2.5 mm, and observation is made using an optical microscope with a visual field of 2.5 mm $\times$ 2.5 mm from a liquid lower surface to a liquid upper surface in increments of 100 $\mu$m. As a result of observing in 10 visual fields and calculating the average number of air bubbles and the average number of insoluble matters, the number of air bubbles is 0.42 pieces/$\mu$L, and the number of insoluble matters is 0 pieces/$\mu$L. After approximately 20 g of the gelatin aqueous solution is poured into each of 14 cylindrical cup-shaped containers made of an aluminum alloy (A5056) that is chamfered to have an inner diameter of 104 mm, a bottom thickness of 5 mm, and a bottom inner circumference of R2 mm and that has an inner surface coated with FEP (NIPPON FUSSO, NF-004A), the gelatin aqueous solution in 14 cylindrical cup-shaped containers is provided on a 350 $\times$ 634 $\times$ 20 mm aluminum plate precooled to approximately -35°C via a glass plate having a thickness of 1 mm, and is allowed to stand for 1 hour with lids closed to obtain a frozen gelatin substance. The linear expansion coefficient of a material (aluminum alloy (A5056)) for the major member of the used cylindrical cup-shaped container is 24.3 $\times$ 10$^{-6}$/K. By freeze-drying the frozen gelatin substance using a freeze-dryer (ULVAC, DFR-5N-B) and removing moisture, a polymer porous substance (freeze-dried substance) is produced.

The polymer porous substance is pulverized with a screen pulverizer (Quadro, Comil U10) using a screen of 0.079 inches and then a screen of 0.040 inches (1 inch is approximately 2.45 cm). A polymer porous substance that passes through a sieve having an opening size of 1,400 $\mu$m and that does not pass through a sieve having an opening size of 300 $\mu$m is obtained.

(Accommodating Step)

The heating treatment apparatus 500 is provided such that the first chamber 10 is on the lower side and the aeration direction is along the vertical direction, and a granular polymer porous substance (heating treatment target object) is accommodated in the first chamber 10 to be at a height of 10 cm from the surface of the holding member 80. The second chamber 20 is attached to the first chamber 10, and the sectionalizing valve 30 is opened.

(Heated Gas Flowing Step)

135°C heated nitrogen is flowed into the container 60 of the heating treatment apparatus 500 via the air supply port 40 and the air exhaust port 50. Simultaneously, the stainless steel plate 70A is heated by the jacket type heater 70B, thereby heating the container 60, and after the temperature of a polymer porous substance reaches 135°C, the temperature is maintained for 5 hours. In this manner, a polymer porous crosslinked substance of Example is produced.

After then, heating of the nitrogen is stopped to continue the flow of the nitrogen, and the jacket type heater is cooled. After the temperature of the polymer porous crosslinked substance becomes 40°C or lower, the polymer porous crosslinked substance is taken out from the first chamber 10. In this case, a total of six locations at level 3 in the height direction and level 2 in a radial direction of the first chamber 10 are randomly selected, and 100 mg of the polymer porous crosslinked substance is collected from each of the six locations.

[Comparative Example]

A granular polymer porous substance (heating treatment target object) is prepared as in Example. The polymer porous substance is pulverized with a screen pulverizer (Quadro, Comil U10) using a screen of 0.079 inches and then a screen of 0.040 inches (1 inch is approximately 2.45 cm). The polymer porous substance that passes through a sieve having an opening of 1, 400 $\mu$m and that does not pass through a sieve having an opening size of 300 $\mu$m is collected, and a 10 mL glass vial is filled with approximately 0.09 g of the polymer porous substance using a filling machine (Aishin Nano Technologies, TF-70AD). The filled vial is provided at a clean oven (Nitto Rika Kogyo, NCO-500A600L-WS), and heating treatment is performed in a nitrogen atmosphere at 135°C for 5 hours to produce a polymer porous crosslinked substance of a comparative example.

An acid residual rate and a water elution component amount are measured using the obtained polymer porous crosslinked substance in the following manner.

[Acid Residual Rate]

The mass of a measuring microtube (trade name: Mini Supertube, manufactured by IBIS Corporation, capacity: 2 ml, and hereinafter referred to as a tube) is measured (A). 15.0 ($\pm$0.2) mg of a polymer porous crosslinked substance is weighed (n = 3), and the measuring tube is filled therewith. 1.7 ml of 1 mol/L HCl is added to the tube in which the polymer porous crosslinked substance is placed, and the mixture is allowed to stand at a constant temperature of 37 $\pm$0.5°C for 3 hours. After a specified time, the tube is placed on ice to stop the reaction, and the mixture is centrifuged at 10,000$\times$g for 1 minute in a centrifuge previously set at 4°C. After confirming that a tissue repair material has settled, a supernatant is absorbed, ultrapure water previously cooled on ice is added, and centrifugation is performed again under the same conditions as described above. Absorbing the supernatant, again adding the ultrapure water, and again performing centrifugation under the same conditions as described above are repeated two times. After absorbing the supernatant, freeze-drying is performed. After taking out from a freeze-dryer, a tube cap is quickly closed in order to prevent the polymer porous crosslinked substance from absorbing moisture in the air. The mass of each tube is measured (C), and the acid residual rate is calculated using the following formula.

$$\text{Acid residual rate} = (C - A)/B \times 100(\%) \cdots (3)$$

In Example using the heating treatment apparatus according to the embodiment of the present disclosure, a polymer porous crosslinked substance, which is a heating treatment object, is accommodated in the first chamber of the container in a state of being sealed with the sectionalizing valve. After the first chamber is connected to an external space via the sectionalizing valve, the heating treatment object can be taken out to the outside from the first chamber by opening the sectionalizing valve. For this reason, the heating treatment object can be taken out from the container while suppressing mixing with foreign substances.

The polymer porous crosslinked substance of Example has an acid residual rate within a range of 45% to 60% at the six locations. In addition, also the polymer porous crosslinked substance of the comparative example has an acid residual rate in a range of 45% to 60%.

Based on the above results, it is confirmed that the heating treatment apparatus according to the embodiment of the present disclosure can collectively perform heat treatment on a large number of granules of a porous polymer and can achieve the same acid residual rate as in the comparative example by using a container having a large capacity compared to the comparative example in which heating treatment is performed using the vial having a small capacity.

The entire disclosure of Japanese Patent Application No. 2021-050325, filed March 24, 2021, is incorporated into the present specification by reference. All documents, patent applications, and technical standards described in the present specification are incorporated into the present specification by reference, as if each of the documents, the patent applications, and the technical standards is specifically and individually described.

**Claims**

1.  A heating treatment apparatus comprising:
    a container that includes a first chamber and a second chamber which are connected to each other via a sectionalizing valve and an air supply port and an air exhaust port through which a heated gas flows.

2.  The heating treatment apparatus according to claim 1,
    wherein the container has a tubular shape.

3.  The heating treatment apparatus according to claim 1 or 2, further comprising:
    a temperature adjusting member that is disposed on an outer side of the container and that is detachably attached to the heating treatment apparatus.

4.  The heating treatment apparatus according to claim 3,
    wherein the temperature adjusting member includes at least one of a jacket type heater or a metal member.

**5.** The heating treatment apparatus according to claim 4,
wherein the metal member is made of stainless steel or is made of aluminum.

**6.** The heating treatment apparatus according to any one of claims 1 to 5,
wherein a holding member that holds a heating treatment target object is provided between the air supply port and the air exhaust port.

**7.** The heating treatment apparatus according to claim 6,
wherein the holding member is a porous plate, a mesh plate, a punching metal, or a breathable bag.

**8.** The heating treatment apparatus according to any one of claims 1 to 7,
wherein the sectionalizing valve is a split butterfly valve.

**9.** A manufacturing method of a heating treatment object comprising:

a step of accommodating a heating treatment target object in the container of the heating treatment apparatus according to any one of claims 1 to 8; and
a step of flowing the heated gas into the container via the air supply port and the air exhaust port.

**10.** The manufacturing method of a heating treatment object according to claim 9,

wherein the heating treatment apparatus further includes a temperature adjusting member, and
the manufacturing method further comprises a step of heating the container with the temperature adjusting member.

**11.** The manufacturing method of a heating treatment object according to claim 10,
wherein the temperature adjusting member includes stainless steel and a heater that heats the stainless steel.

**12.** The manufacturing method of a heating treatment object according to claim 10 or 11,
wherein at least a part of the step of flowing the heated gas and at least a part of the step of heating the container are simultaneously performed.

**13.** The manufacturing method of a heating treatment object according to any one of claims 9 to 12,
wherein the heating treatment target object is a polymer porous substance of collagen or gelatin.

**14.** The manufacturing method of a heating treatment object according to any one of claims 9 to 13,
wherein the heated gas is an inert gas.

**15.** The manufacturing method of a heating treatment object according to any one of claims 9 to 14, further comprising:
a step of collecting a heating treatment object by closing the sectionalizing valve after the step of flowing the heated gas.

# FIG. 1

# FIG. 2

# FIG. 3

# FIG. 4

# FIG. 5

# FIG. 6

# FIG. 7

## INTERNATIONAL SEARCH REPORT

| | International application No. |
|---|---|
| | **PCT/JP2022/012436** |

**A. CLASSIFICATION OF SUBJECT MATTER**

*F27B 1/02*(2006.01)i; *F27D 7/02*(2006.01)i; *F24H 3/00*(2022.01)i
FI: F27B1/02; F27D7/02 Z; F24H3/00 B

According to International Patent Classification (IPC) or to both national classification and IPC

**B. FIELDS SEARCHED**

Minimum documentation searched (classification system followed by classification symbols)

F24H3/00; F27B1/02; F27D7/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2019/189411 A1 (TOHO TITANIUM CO., LTD.) 03 October 2019 (2019-10-03) paragraphs [0022]-[0026], fig. 3 | 1-15 |
| A | JP 62-7432 A (D'AGROSA, David D.) 14 January 1987 (1987-01-14) page 4, upper left column, lines 3-10, fig. 1 | 1-15 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **18 April 2022** | **10 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

International application No.

**PCT/JP2022/012436**

| Patent document cited in search report | | | Publication date (day/month/year) | Patent family member(s) | | | Publication date (day/month/year) |
|---|---|---|---|---|---|---|---|
| WO | 2019/189411 | A1 | 03 October 2019 | CN | 111936424 | A | |
| | | | | paragraphs [0035]-[0039], fig. 3 | | | |
| | | | | KR | 2020/0131875 | A | |
| JP | 62-7432 | A | 14 January 1987 | US | 4740157 | A | |
| | | | | page 5, lines 26-37, fig. 1 | | | |
| | | | | CN | 086103744 | A | |
| | | | | page 6, lines 22-24, page 7, lines 1-6, fig. 1 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014141877 A
- EP 1014176 B
- US 6992172 B
- WO 200485473 A
- WO 2008103041 A
- JP 2010519293 A
- JP 2010519252 A
- JP 2010518833 A
- JP 2010519251 A
- WO 2010128672 A
- WO 2010147109 A
- JP 2021050325 A